# EUROPÄISCHE PATENTSCHRIFT

(11) **EP 2 283 767 B1**
(45) Veröffentlichungstag und Bekanntmachung des Hinweises auf die Patenterteilung: **20.07.2016**
(21) Anmeldenummer: 10008132.2
(22) Anmeldetag: 04.08.2010
(51) Int. Cl.: A61B 1/00, A61B 1/07

(54) **Endoskop**
Endoscope
Endoscope

(30) Priorität: 14.08.2009 DE 102009037318
(43) Veröffentlichungstag der Anmeldung: 16.02.2011
(73) Patentinhaber: Karl Storz GmbH & Co. KG, 78532 Tuttlingen (DE)
(72) Erfinder: Dahmen, Jan, 78532 Tuttlingen (DE); Teichtmann, Elmar, 75031 Eppingen (DE)

(56) Entgegenhaltungen:
- EP-A1- 0 460 829
- DE-A1- 19 713 275
- DE-U- 7 520 162
- US-A- 2 975 785
- US-A- 4 390 012

## Beschreibung

Die Erfindung betrifft ein Endoskop mit einem Instrumentengehäuse und mit einem am distalen Ende des Instrumentengehäuses angeordneten Schaft, der aus einem Außenrohr und einem zumindest teilweise in dem Außenrohr angeordneten Innenrohr besteht, wobei das Außenrohr und das Innenrohr in Axialrichtung des Schaftes relativ zueinander bewegbar am Instrumentengehäuse gelagert sind.

Derartige Endoskope sind aus der Praxis allgemein bekannt. Das Innenrohr des Schaftes dient zur Aufnahme optischer Bauteile, wie beispielsweise Stablinsen. Zwischen dem Innenrohr und dem das Innenrohr umgebenden Außenrohr des Schaftes ist in der Regel ein freier Ringraum ausgebildet, der beispielsweise zur Aufnahme von Lichtleiterfasern dient, um Licht zum distalen Ende des Endoskops und somit zum zu beobachtenden Operationsgebiet zu leiten.

Nach jedem Gebrauch müssen die Endoskope sterilisiert werden, wozu sie in Autoklaven feuchter Hitze im Temperaturbereich von 120-140°C ausgesetzt werden. Aufgrund unterschiedlicher Materialien der beiden Rohre des Instrumentenschaftes in Verbindung mit Temperaturschwankungen und/oder aufgrund unterschiedlicher Temperaturen von Außenrohr und Innenrohr, bedingt durch eine Verzögerung bei der Wärmeleitung vom Außenrohr zum Innenrohr dehnen sich das Außenrohr und das Innenrohr bei diesen, z.T. extremen, Temperaturbelastungen unterschiedlich aus. Um daraus resultierende Spannungen zwischen den distalseitig miteinander verbundenen Rohren des Instrumentenschaftes abbauen zu können, sind das Außenrohr und das Innenrohr in Axialrichtung des Schaftes relativ zueinander bewegbar am Instrumentengehäuse gelagert.

Ein gattungsgemäßes Endoskop mit Längenausgleich bei thermischer Belastung ist beispielsweise aus der DE 197 13 275 A1 bekannt. Bei dieser bekannten Konstruktion sind das Außenrohr und das Innenrohr des Schaftes jeweils fluiddicht eine Baueinheit bildend proximalseitig jeweils mit einem separaten Gehäuseteil des Instrumentengehäuses verbunden. Zur Ermöglichung der erforderlichen axialen Relativbewegung zwischen dem Außenrohr und dem Innenrohr sind die beiden separaten Gehäuseteile über zwischengelagerte O-Ringe schwimmend aneinander gelagert.

Diese bekannte Konstruktion hat sich durchaus bewährt und ermöglicht einen ausreichenden thermischen Längenausgleich der Schaftrohre, jedoch gewährleisten die O-Ringe insbesondere bei der hohen thermischen Belastung während der Reinigung des Endoskops keine dauerhafte fluiddichte Abdichtung des Instrumentengehäuses.

Davon ausgehend liegt der Erfindung die **Aufgabe** zugrunde, ein Endoskop zu schaffen, das eine dauerhaft fluiddichte Relativbewegung der beiden Schaftrohre zueinander gewährleistet.

Die **Lösung** dieser Aufgabenstellung ist erfindungsgemäß dadurch gekennzeichnet, dass die in Axialrichtung des Schaftes relativbewegliche Lagerung des Außenrohres und des Innenrohres am Instrumentengehäuse mittels mindestens einer flexiblen Membran erfolgt.

Durch den erfindungsgemäßen Einsatz mindestens einer Membran zwischen den miteinander zu verbindenden Bauteilen wird aufgrund der Flexibilität der Membran die Relativbewegung zwischen den beiden Rohren des Instrumentenschaftes ermöglicht und gleichzeitig eine flexible Verbindung geschaffen, die auch bei häufigem Gebrauch eine dauerhaft fluiddichte Verbindung gewährleistet.

Gemäß einer bevorzugten Ausführungsform der Erfindung wird vorgeschlagen, dass die mindestens eine flexible Membran eine Metallmembran ist. Korrosionsfeste Metallmembranen zeichnen sich dadurch aus, dass sie auch bei häufigem Gebrauch und vielen Reinigungsprozessen mit hohen Temperaturbelastungen dauerhaft beständig sind.

Mit einer praktischen Ausführungsform der Erfindung wird vorgeschlagen, dass das Instrumentengehäuse einen mit dem proximalen Ende des Außenrohres verbundenen äußeren Gehäuseteil und einen mit dem proximalen Ende des Innenrohres verbundenen inneren Gehäuseteil umfasst. Die Aufteilung des Instrumentengehäuses in einen äußeren und einen inneren Gehäuseteil ermöglicht eine besonders variable Ausgestaltung zur Erzielung der gewünschten Relativbeweglichkeit zwischen den beiden Rohren des Instrumentenschaftes.

Gemäß einer ersten Ausführungsform der Erfindung wird vorgeschlagen, dass das Außenrohr und der äußere Gehäuseteil über die mindestens eine flexible Membran miteinander verbunden sind. Bei dieser Ausgestaltungsform erlaubt die Membranverbindung zwischen dem äußeren Gehäuseteil und dem Außenrohr aufgrund ihrer Flexibilität die temperaturbedingte Längenänderung des Außenrohres und die damit verbundene Relativbewegung zum Innenrohr.

Gemäß einer zweiten Ausführungsform der Erfindung wird vorgeschlagen, dass das Innenrohr und der innere Gehäuseteil über die mindestens eine flexible Membran miteinander verbunden sind. Diese Ausgestaltungsform kann alternativ oder zusätzlich zu der Anordnung einer Membran zwischen dem äußeren Gehäuseteil und dem Außenrohr angewendet werden.

Mit einer dritten erfindungsgemäßen Ausführungsform wird vorgeschlagen, dass der äußere Gehäuseteil und der innere Gehäuseteil über die mindestens eine flexible Membran miteinander verbunden sind. Bei dieser Ausgestaltungsform wird die Relativbeweglichkeit zwischen den beiden Rohren des Instrumentenschaftes dadurch ermöglicht, dass die beiden Rohre jeweils fluiddicht und fest mit ihren zugehörigen Gehäuseteilen verbunden sind und die flexible Membran zwischen den beiden mit jeweils einem Rohr versehenen Gehäuseteilen angeordnet ist. Diese Ausführungsform ermöglicht die Montage der fertigen Baueinheiten Außenrohr - äußeres Gehäuseteil einerseits und Innenrohr - inneres Gehäuseteil andererseits, die über die flexible Membran zwischen dem äußeren Gehäuseteil und dem inneren Gehäuseteil miteinander verbunden werden.

Die Anordnung der Membran zwischen dem äußeren Gehäuseteil und dem Außenrohr erfolgt gemäß einer ersten praktischen Ausführungsform der Erfindung am distalen Ende des am Außenrohr anliegenden Bereichs des äußeren Gehäuseteils. Gemäß einer alternativen Ausführungsform zur Anordnung der Membran zwischen dem äußeren Gehäuseteil und dem Außenrohr wird vorgeschlagen, dass die Membran am proximalen Ende des am Außenrohr anliegenden Bereichs des äußeren Gehäuseteils angeordnet ist.

Um insbesondere bei der Anordnung der flexiblen Membran weiter innenliegend am Instrumentengehäuse das Eindringen von Feuchtigkeit und/oder Schmutz zwischen die miteinander zu verbindenden Bauteile zu verhindern, wird erfindungsgemäß weiterhin vorgeschlagen, dass zusätzlich zu der mindestens einen Membran mindestens ein Dichtungselement zwischen den über die Membran miteinander verbundenen Bauteilen angeordnet ist, wobei das Dichtungselement vorzugsweise als O-Ring ausgebildet ist.

Gemäß einer bevorzugten Ausführungsform der Erfindung wird vorgeschlagen, dass die mindestens eine Membran als einstückig mit dem Instrumentengehäuse gefertigter dünnwandiger Gehäuseteil ausgebildet ist. Durch diese einstückige Ausbildung der Membran als Teil des Instrumentengehäuses entfällt gehäuseseitig ein fluiddichte Abdichtung zum anzuschließenden Bauteil, wie sie am freien anderen Ende der Membran beispielsweise durch Verlöten, Verkleben oder Verschweißen auszubilden ist.

Schließlich wird mit der Erfindung vorgeschlagen, dass die mindestens eine Membran als separates Bauteil ausgebildet ist, das fluiddicht an den miteinander zu verbindenden Bauteilen festlegbar ist. Durch die Ausbildung der Membran als separates Bauteil ist es möglich, durch geeignete Materialwahl, das heißt, unabhängig vom Material des Instrumentengehäuses, das für den Einzelfall geeignetste Material auszuwählen, das die gewünschte Relativbewegung zwischen den miteinander zu verbindenden Bauteilen ermöglicht.

Weitere Merkmale und Vorteile der Erfindung ergeben sich anhand der zugehörigen Zeichnungen, in denen vier Ausführungsbeispiele eines erfindungsgemäßen Endoskops nur beispielhaft dargestellt sind, ohne die Erfindung auf diese Ausführungsbeispiele zu beschränken. In den Zeichnungen zeigt:
- Fig. 1: eine schematische Seitenansicht eines erfindungsgemäßen Endoskops;
- Fig. 2: eine vergrößerte Ansicht des Details II gemäß Fig. 1, eine erste Ausführungsform darstellend;
- Fig. 3: eine vergrößerte Ansicht des Details III gemäß Fig. 1, eine zweite Ausführungsform darstellend;
- Fig. 4: eine vergrößerte Ansicht des Details IV gemäß Fig. 1, eine dritte Ausführungsform darstellend und
- Fig. 5: eine vergrößerte Ansicht des Details V gemäß Fig. 1, eine vierte Ausführungsform darstellend.

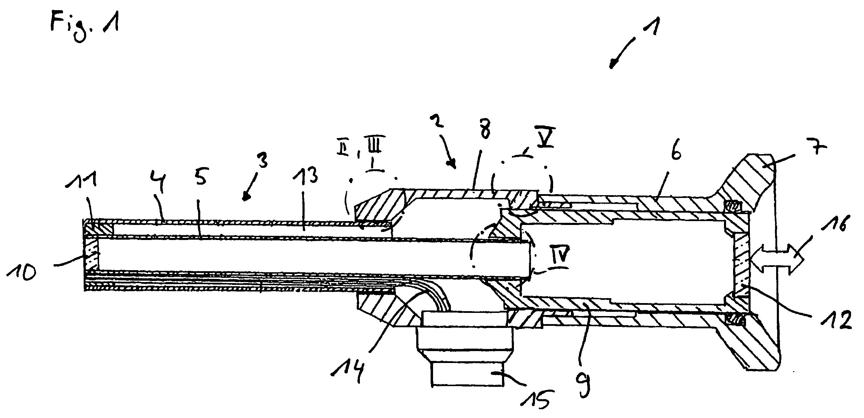

Die Abbildung Fig. 1 zeigt ein Endoskop 1, das im Wesentlichen aus einem Instrumentengehäuse 2 und einem am distalen Ende des Instrumentengehäuses 2 angeordneten hohlen Schaft 3 besteht, der seinerseits aus einem hohlen Außenrohr 4 und einem in dem Außenrohr 4 angeordneten Innenrohr 5 besteht. Am proximalen Ende des Instrumentengehäuses 2 ist ein Optikkopf 6 mit einer Okularmuschel 7 angeordnet.

Bei der dargestellten Ausführungsform besteht auch das Instrumentengehäuse 2 aus zwei Teilen, nämlich einem mit dem proximalen Ende des Außenrohres 4 verbundenen äußeren Gehäuseteil 8 und einem mit dem proximalen Ende des Innenrohres 5 verbundenen inneren Gehäuseteil 9.

Das Innenrohr 5 des Schaftes 3 dient zur Aufnahme optischer Bauteile, wie beispielsweise Stablinsen. Aus Gründen einer besseren Übersichtlichkeit der Darstellung wurden diese im Inneren des Innenrohres 5 angeordneten optischen Bauteile nicht dargestellt. Distalseitig ist das Innenrohr 5 mit einer durchsichtigen Scheibe 10 fluiddicht verschlossen und über eine beispielsweise ringförmig ausgebildete Dichtung 11 fluiddicht mit dem distalen Ende des Außenrohres 4 verbunden.

Proximalseitig ist das dargestellte Instrumentengehäuse 2 durch eine fluiddicht im proximalen Ende des inneren Gehäuseteil 9 angeordnete durchsichtige Scheibe 12 verschlossen.

Durch die im Wesentlichen koaxiale Anordnung von Außenrohr 4 und Innenrohr 5 ist zwischen der Innenseite des Außenrohres 4 und der Außenseite des Innenrohres 5 ein Ringraum 13 ausgebildet, der beispielsweise zur Aufnahme von Lichtleiterfasern 14 dient, die über einen Stutzen 15 im Instrumentengehäuse 2 bis an das distale Ende des Schaftes 3 geführt werden, um Licht zum distalen Ende des Endoskops 1 und somit zum zu beobachtenden Operationsgebiet zu leiten. In der Dichtung 11 sind dann entsprechende Lichtaustrittsöffnungen ausgebildet.

Da ein Endoskop 1 nach jedem Gebrauch sterilisiert werden muss, wird es in einem Autoklaven feuchter Hitze im Temperaturbereich von 120-140°C ausgesetzt. Dieses Aufheizen des Endoskops 1 und das abschließende Abkühlen hat aufgrund unterschiedlicher Materialien der beiden Rohre 4 und 5 des Schaftes 3 in Verbindung mit Temperaturschwankungen und/oder aufgrund unterschiedlicher Temperaturen von Außenrohr 4 und Innenrohr 5, bedingt durch eine Verzögerung bei der Wärmeleitung vom Außenrohr 4 zum Innenrohr 5 zur Folge, dass sich das Außenrohr 4 und das Innenrohr 5 bei diesen, z.T. extremen, Temperaturbelastungen unterschiedlich ausdehnen. Um daraus resultierende Spannungen zwischen den distalseitig miteinander verbundenen Rohren 4 und 5 des Schaftes 3 abbauen zu können, sind das Außenrohr 4 und das Innenrohr 5 in Axialrichtung des Schaftes 3 relativ zueinander bewegbar am Instrumentengehäuse 2 gelagert, wie dies durch den Doppelpfeil 16 gemäß Fig. 1 dargestellt ist.

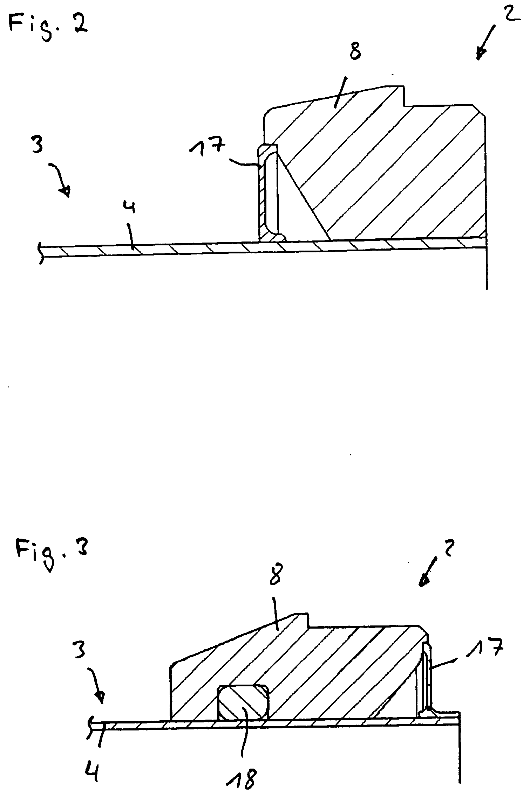

Wie aus den Abbildungen Fig. 2 bis 5 ersichtlich, wird die in Axialrichtung des Schaftes 3 relativbewegliche Lagerung des Außenrohres 4 und des Innenrohres 5 am Instrumentengehäuse 3 mittels mindestens einer flexiblen Membran 17 ermöglicht, die vorzugsweise als korrosionsbeständige Metallmembran ausgebildet ist.

Bei der in Fig. 2 dargestellten ersten Ausführungsform verbindet die Membran 17 das Außenrohr 4 und den äußeren Gehäuseteil 8 und ermöglicht so aufgrund der Flexibilität der Membran 17 eine Relativbewegung zwischen dem Außenrohr 4 und dem Instrumentengehäuse 2 und damit zwischen dem Außenrohr 4 und dem Innenrohr 5. Bei dieser dargestellten Ausführungsform ist die Membran 17 am distalen Ende des am Außenrohr 4 anliegenden Bereichs des äußeren Gehäuseteils 8 angeordnet.

Bei der alternativen zweiten Ausführungsform gemäß Fig. 3 verbindet die Membran 17 wiederum das Außenrohr 4 und den äußeren Gehäuseteil 8 und ermöglicht so aufgrund der Flexibilität der Membran 17 eine Relativbewegung zwischen dem Außenrohr 4 und dem Instrumentengehäuse 2 und damit zwischen dem Außenrohr 4 und dem Innenrohr 5, jedoch ist die Membran 17 bei dieser Ausführungsform am proximalen Ende des am Außenrohr 4 anliegenden Bereichs des äußeren Gehäuseteils 8 angeordnet.

Die Verbindung der Membran 17 mit den miteinander zu verbindenden Bauteilen Außenrohr 4 und äußerer Gehäuseteil 8 erfolgt in beiden Fällen fluiddicht, beispielsweise durch Verlöten, Verkleben oder Verschweißen.

Um insbesondere bei der Anordnung der flexiblen Membran 17 weiter innenliegend am Instrumentengehäuse, wie dies in Fig. 3 dargestellt ist, das Eindringen von Feuchtigkeit und/oder Schmutz zwischen die miteinander zu verbindenden Bauteile 4 und 8 zu verhindern, ist bei der dargestellten Ausführungsform zusätzlich zu der Membran 17 ein Dichtungselement 18 zwischen den über die Membran 17 miteinander verbundenen Bauteilen Außenrohr 4 und äußerer Gehäuseteil 8 angeordnet, wobei das Dichtungselement 18 als O-Ring ausgebildet ist.

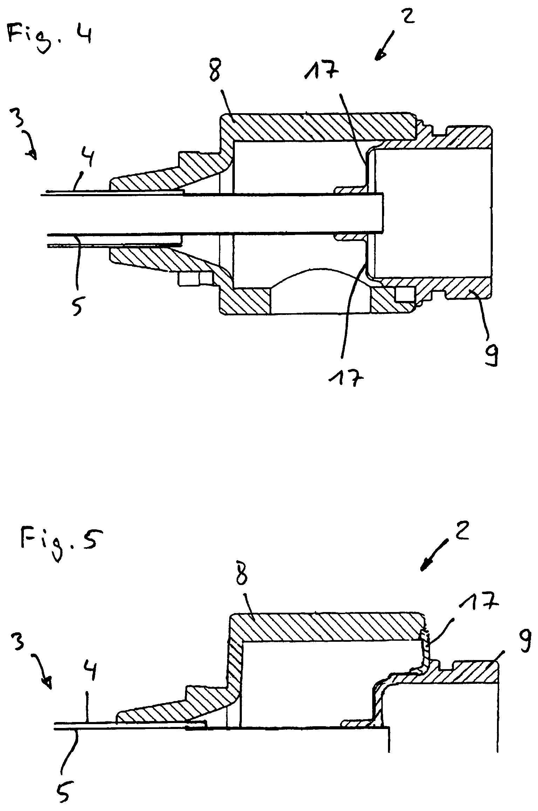

Die Abbildung Fig. 4 zeigt eine dritte Ausführungsform zur Anordnung der Membran 17. Bei dieser Ausführungsform verbindet die Membran 17 das Innenrohr 5 und den inneren Gehäuseteil 9 und ermöglicht so aufgrund der Flexibilität der Membran 17 eine Relativbewegung zwischen dem Innenrohr 5 und dem Instrumentengehäuse 2 und damit zwischen dem Innenrohr 5 und dem Außenrohr 4.

Die Ausführungsformen gemäß Fig. 2 und 4 oder Fig. 3 und 4 können alternativ oder auch zusammen in einer Konstruktion verwirklicht werden, so dass bei der Addition der beiden Ausführungsformen das Außenrohr 4 und der äußere Gehäuseteil 8 sowie das Innenrohr 5 und der innere Gehäuseteil 9 über jeweils eine flexible Membran 17 miteinander verbunden sind.

Fig. 5 zeigt schließlich eine alternative vierte Ausführungsform zur Anordnung der Membran 17 am Instrumentengehäuse 2. Anders als bei den zuvor vorgestellten Ausführungsformen verbindet mindestens eine flexible Membran 17 bei dieser Ausgestaltungsform nicht nur das Instrumentengehäuse 2 direkt mit einem der beiden Rohre 4 und/oder 5 des Schaftes 6, sondern auch den äußeren Gehäuseteil 8 und den inneren Gehäuseteil 9 des Instrumentengehäuse 2.

Bei dieser Ausgestaltungsform sind das Außenrohr 4 und der äußere Gehäuseteil 8 sowie das Innenrohr 5 und der innere Gehäuseteil 9 jeweils fluiddicht zu einer Einheit miteinander verbunden. Die Verbindung des äußeren Gehäuseteils 8 und des inneren Gehäuseteils 9 über die Membran 17 ermöglicht so aufgrund der Flexibilität der Membran 17 und der festen Einbindung der Rohre 4 und 5 in ihren jeweiligen Gehäuseteilen 8 und 9 eine Relativbewegung zwischen dem Innenrohr 5 und dem Außenrohr 4.

Auch für die in Fig. 4 dargestellten Ausführungsformen gilt, dass die Verbindung der Membran 17 mit den miteinander zu verbindenden Bauteilen Innenrohr 5 und innerer Gehäuseteil 9 bzw. äußerer Gehäuseteil 8 und innerer Gehäuseteil 9, beispielsweise durch Verlöten, Verkleben oder Verschweißen, fluiddicht ausgebildet ist.

Während bei den in Fig. 2, 3 und 5 dargestellten Ausführungsformen die flexible Membran 17 als separates Bauteil ausgebildet ist, das, beispielsweise durch Verlöten, Verkleben oder Verschweißen, fluiddicht an den beiden miteinander zu verbindenden Bauteilen festgelegt wird, zeigt die Abbildung Fig. 4 eine Ausführungsform, bei der die Membran 17 einseitig einstückig mit dem inneren Gehäuseteil 9 gefertigter dünnwandiger Gehäuseteil ausgebildet ist, so dass es zum inneren Gehäuseteil keiner besonderen fluiddichten Abdichtung bedarf.

Ein wie zuvor beschrieben ausgestaltetes Endoskop 1 zeichnet sich dadurch aus, dass es durch die Anordnung der mindestens einen flexiblen Membran 17 bei einfachem Aufbau eine dauerhaft fluiddichte Relativbewegung der beiden Rohre 4 und 5 des Schaftes 3 zueinander gewährleistet.

**Bezugszeichenliste**

| | | | |
|---|---|---|---|
| 1 | Endoskop | 18 | Dichtungselement |
| 2 | Instrumentengehäuse | | |
| 3 | Schaft | | |
| 4 | Außenrohr | | |
| 5 | Innenrohr | | |
| 6 | Optikkopf | | |
| 7 | Okularmuschel | | |
| 8 | äußerer Gehäuseteil | | |
| 9 | innerer Gehäuseteil | | |
| 10 | Scheibe | | |
| 11 | Dichtung | | |
| 12 | Scheibe | | |
| 13 | Ringraum | | |
| 14 | Lichtleiterfasern | | |
| 15 | Stutzen | | |
| 16 | Doppelpfeil / axiale Längenausdehnung | | |
| 17 | Membran | | |

## Patentansprüche

1. Endoskop mit einem Instrumentengehäuse (2) und mit einem am distalen Ende des Instrumentengehäuses (2) angeordneten Schaft (3), der aus einem Außenrohr (4) und einem zumindest teilweise in dem Außenrohr (4) angeordneten Innenrohr (5) besteht, wobei das Außenrohr (4) und das Innenrohr (5) in Axialrichtung des Schaftes (3) relativ zueinander bewegbar am Instrumentengehäuse (2) gelagert sind,
**dadurch gekennzeichnet,**
**dass** die in Axialrichtung des Schaftes (3) relativbewegliche Lagerung des Außenrohres (4) und des Innenrohres (5) mittels mindestens einer flexiblen Membran (17) erfolgt, die das Instrumentengehäuse (2) direkt mit zumindest einem der beiden Rohre (4,5) des Schaftes verbindet.

2. Endoskop nach Anspruch 1, **dadurch gekennzeichnet, dass** die mindestens eine flexible Membran (17) eine Metallmembran ist.

3. Endoskop nach Anspruch 1 oder 2, **dadurch gekennzeichnet, dass** das Instrumentengehäuse (2) einen mit dem proximalen Ende des Außenrohres (4) verbundenen äußeren Gehäuseteil (8) und einen mit dem proximalen Ende des Innenrohres (5) verbundenen inneren Gehäuseteil (9) umfasst.

4. Endoskop nach Anspruch 3, **dadurch gekennzeichnet, dass** das Außenrohr (4) und der äußere Gehäuseteil (8) über die mindestens eine flexible Membran (17) miteinander verbunden sind.

5. Endoskop nach Anspruch 3, **dadurch gekennzeichnet, dass** das Innenrohr (5) und der innere Gehäuseteil (9) über die mindestens eine flexible Membran (17) miteinander verbunden sind.

6. Endoskop nach Anspruch 3, **dadurch gekennzeichnet, dass** der äußere Gehäuseteil (8) und der innere Gehäuseteil (9) über die mindestens eine flexible Membran (17) miteinander verbunden sind.

7. Endoskop nach Anspruch 3, **dadurch gekennzeichnet, dass** das Außenrohr (4) und der äußere Gehäuseteil (8) sowie das Innenrohr (5) und der innere Gehäuseteil (9) über jeweils mindestens eine flexible Membran (17) miteinander verbunden sind.

8. Endoskop nach Anspruch 4, **dadurch gekennzeichnet, dass** die Membran (17) am distalen Ende des am Außenrohr (4) anliegenden Bereichs des äußeren Gehäuseteils (8) angeordnet ist.

9. Endoskop nach Anspruch 4, **dadurch gekennzeichnet, dass** die Membran (17) am proximalen Ende des am Außenrohr (4) anliegenden Bereichs des äußeren Gehäuseteils (8) angeordnet ist.

10. Endoskop nach einem der Ansprüche 3 bis 9, **dadurch gekennzeichnet, dass** zusätzlich zu der mindestens einen Membran (17) mindestens ein Dichtungselement (18) zwischen den über die Membran (17) miteinander verbundenen Bauteilen angeordnet ist.

11. Endoskop nach Anspruch 10, **dadurch gekennzeichnet, dass** das mindestens eine Dichtungselement (18) als O-Ring ausgebildet ist.

12. Endoskop nach einem der Ansprüche 1 bis 11, **dadurch gekennzeichnet, dass** die mindestens eine Membran (17) als einstückig mit dem Instrumentengehäuse (2) gefertigter dünnwandiger Gehäuseteil ausgebildet ist.

13. Endoskop nach Anspruch 12, **dadurch gekennzeichnet, dass** die mindestens eine Membran (17) einseitig einstückig mit dem Instrumentengehäuse (2) ausgebildet ist und mit dem freien anderen Ende fluiddicht an einem anderen Bauteil festgelegt ist.

14. Endoskop nach einem der Ansprüche 1 bis 11, **dadurch gekennzeichnet, dass** die mindestens eine Membran (17) als separates Bauteil ausgebildet ist, das fluiddicht an den miteinander zu verbindenden Bauteilen festlegbar ist.

## Claims

1. Endoscope with an instrument housing (2) and with a shaft (3) arranged at the distal end of the instrument housing (2), which shaft (3) consists of an outer tube (4) and of an inner tube (5) arranged at least partially in the outer tube (4), said outer tube (4) and said inner tube (5) being mounted on the instrument housing (2) so as to be movable relative to each other in the axial direction of the shaft (3),
**characterized in that**
the mounting of the outer tube (4) and the inner tube (5) with relative mobility in the axial direction of the shaft (3) is effected by means of at least one flexible membrane (17), which connects the instrument housing (2) directly to at least one of the two tubes (4, 5) of the shank.

2. Endoscope according to Claim 1, **characterized in that** the at least one flexible membrane (17) is a metal membrane.

3. Endoscope according to Claim 1 or 2, **characterized in that** the instrument housing (2) comprises an outer housing part (8), connected to the proximal end of the outer tube (4), and an inner housing part (9), connected to the proximal end of the inner tube (5).

4. Endoscope according to Claim 3, **characterized in that** the outer tube (4) and the outer housing part (8) are connected to each other by the at least one flexible membrane (17).

5. Endoscope according to Claim 3, **characterized in that** the inner tube (5) and the inner housing part (9) are connected to each other by the at least one flexible membrane (17).

6. Endoscope according to Claim 3, **characterized in that** the outer housing part (8) and the inner housing part (9) are connected to each other by the at least one flexible membrane (17).

7. Endoscope according to Claim 3, **characterized in that** the outer tube (4) and the outer housing part (8) and also the inner tube (5) and the inner housing part (9) are connected to each other in each case by at least one flexible membrane (17).

8. Endoscope according to Claim 4, **characterized in that** the membrane (17) is arranged at the distal end of the area of the outer housing part (8) bearing on the outer tube (4).

9. Endoscope according to Claim 4, **characterized in that** the membrane (17) is arranged at the proximal end of the area of the outer housing part (8) bearing on the outer tube (4).

10. Endoscope according to one of Claims 3 to 9, **characterized in that**, in addition to the at least one membrane (17), at least one sealing element (18) is arranged between the components that are connected to each other by the membrane (17).

11. Endoscope according to Claim 10, **characterized in that** the at least one sealing element (18) is designed as an 0-ring.

12. Endoscope according to one of Claims 1 to 11, **characterized in that** the at least one membrane (17) is designed as a thin-walled housing part produced in one piece with the instrument housing (2).

13. Endoscope according to Claim 12, **characterized in that** the at least one membrane (17) is, on one side, designed in one piece with the instrument housing (2) and, with the free other end, is secured in a fluid-tight manner on another component.

14. Endoscope according to one of Claims 1 to 11, **characterized in that** the at least one membrane (17) is designed as a separate component which can be secured in a fluid-tight manner on the components that are to be connected to each other.

## Revendications

1. Endoscope avec un carter d'instrument (2) et avec un manche (3) disposé au niveau de l'extrémité distale du carter d'instrument (2) et composé d'un tube extérieur (4) et d'un tube intérieur (5) disposé au moins en partie dans le tube extérieur (4), le tube extérieur (4) et le tube intérieur (5) étant disposés dans la direction axiale du manche (3) de façon mobile l'un par rapport à l'autre au niveau du carter d'instrument (2), **caractérisé en ce que** le positionnement relativement mobile du tube extérieur (4) et du tube intérieur (5) s'effectue dans la direction axiale du manche (3) à l'aide d'au moins une membrane flexible (17) reliant le carter d'instrument (2) directement à au moins un des deux tubes (4, 5) du manche.

2. Endoscope selon la revendication 1, **caractérisé en ce que** l'au moins une membrane (17) flexible est une membrane métallique.

3. Endoscope selon la revendication 1 ou 2, **caractérisé en ce que** le carter d'instrument (2) comprend une partie de carter extérieure (8) reliée à l'extrémité proximale du tube extérieur (4) et une partie de carter intérieure (9) reliée à l'extrémité proximale du tube intérieur (5).

4. Endoscope selon la revendication 3, **caractérisé en ce que** le tube extérieur (4) et la partie de carter extérieure (8) sont reliés entre eux via l'au moins une membrane (17) flexible.

5. Endoscope selon la revendication 3, **caractérisé en ce que** le tube intérieur (5) et la partie de carter intérieure (9) sont reliés entre eux via l'au moins une membrane (17) flexible.

6. Endoscope selon la revendication 3, **caractérisé en ce que** la partie de carter extérieure (8) et la partie de carter intérieure (9) sont reliées entre elles via l'au moins une membrane (17) flexible.

7. Endoscope selon la revendication 3, **caractérisé en ce que** le tube extérieur (4) et la partie de carter extérieure (8) ainsi que le tube intérieur (5) et la partie de carter intérieure (9) sont reliés entre eux via respectivement au moins une membrane (17) flexible.

8. Endoscope selon la revendication 4, **caractérisé en ce que** la membrane (17) est disposée au niveau de l'extrémité distale de la région, reposant contre le tube extérieur (4), de la partie de carter extérieure (8).

9. Endoscope selon la revendication 4, **caractérisé en ce que** la membrane (17) est disposée au niveau de l'extrémité proximale de la région, reposant contre le tube extérieur (4), de la partie de carter extérieure (8).

10. Endoscope selon l'une quelconque des revendications 3 à 9, **caractérisé en ce qu'**en sus de l'au moins une membrane (17), au moins un élément d'étanchéité (18) est disposé entre les composants reliés entre eux via la membrane (17).

11. Endoscope selon la revendication 10, **caractérisé en ce que** l'au moins un élément d'étanchéité (18) est réalisé sous la forme d'un joint torique.

12. Endoscope selon l'une quelconque des revendications 1 à 11, **caractérisé en ce que** l'au moins une membrane (17) est réalisée sous la forme d'une partie de carter à paroi mince fabriquée d'un seul tenant avec le carter d'instrument (2).

13. Endoscope selon la revendication 12, **caractérisé en ce que** l'au moins une membrane (17) est réalisée d'un côté d'un seul tenant avec le carter d'instrument (2) et est fixée avec l'autre extrémité libre à un autre composant, de façon étanche aux fluides.

14. Endoscope selon l'une quelconque des revendications 1 à 11, **caractérisé en ce que** l'au moins une membrane (17) est réalisée sous la forme d'un composant séparé pouvant être fixé de façon étanche aux fluides aux composants à relier entre eux.
